# EUROPEAN PATENT APPLICATION

(11) **EP 3 815 546 A1**
(43) Date of publication of application: **05.05.2021**
(21) Application number: 19206271.9
(22) Date of filing: 30.10.2019
(51) Int. Cl.: A23L 33/14, A61K 8/97, A61K 35/74, A61P 35/00

(54) **MEDICAL COMPOSITIONS AND THEIR USE IN TREATING PANCREATIC CANCER**

(71) Applicant: Biomillenia SAS, 93230 Romainville (FR)
(72) Inventor: Dajkovic, Aleksander, 75018 Paris (DE); Loeffert, Dirk, 42781 Haan (DE)
(74) Representative: CH Kilger Anwaltspartnerschaft mbB

(57) **Abstract**

The invention relates to a pharmaceutical composition comprising (i) a live strain from the genus of Kazachstania spp, Acetobacter spp. and/or Stenotrophomonas spp, (ii) an attenuated strain from the genus of Kazachstania spp, Acetobacter spp. and/or Stenotrophomonas spp or, (iii) a medically active substance isolated from a strain from the genus of Kazachstania spp, Acetobacter spp. and/or Stenotrophomonas spp. These may be used to treat pancreatic cancer.

## Description

### FIELD OF THE INVENTION

The invention is in the field of strain and compound development of microorganisms. It relates to the production and/or selection of microorganisms and/or derivatives or compounds thereof, and their use in the treatment, prevention or therapy of diseases related to among others dysbiosis of Malassezia spp. The diseases might be diseases such as inflammatory diseases and/or skin diseases and/or oncogenesis. The present invention provides antimicrobial and/or fungicide for Malassezia spp., therefore providing treatment/prevention/therapy options for diseases related to the presence of Malassezia spp.

### BACKGROUND

It has been noted in recent years that dysbiosis of the microbiome effects the health condition of an organism and might be involved in many diseases such as inflammatory diseases of the digestive system such as IBD (inflammatory bowel disease or inflammatory bowel syndrome, ulcerative colitis, Crohn's disease), metabolic diseases such as diabetes, metabolic syndrome, NASH, obesity, or diseases of the skin and scalp such as psorasis, acne, dandruff as well as being involved in oncogenesis to name a few examples only. It has also been noted that commensal microorganisms that may play an important role in inflammatory responses of the immune system not only at their natural body niche but also in body niches where these commensal microorganisms are commonly strongly underrepresented or not present.

One skin commensal fungal genus, the fungus Malassezia (Malassezia restricta, Malassezia globosa) has been already described to be closely associated with seborrheic dermatitis (SD) and dandruff that are common dermatological problems that affect the seborrheic areas of the body. They are considered the same basic condition sharing many features and responding to similar treatments, differing only in locality and severity. Dandruff is restricted to the scalp, and involves itchy, flaking skin without visible inflammation. SD affects the scalp as well as face, retro-auricular area, and the upper chest, causing flaking, scaling, inflammation and pruritus, and can have marked erythema. Flaking in SD and dandruff is usually white-to-yellowish and may be oily or dry. It is estimated that SD and dandruff combined affect half of the adult population. Recently, it has been also reported that Malassezia restricta appears to be linked with a severe chronic inflammation of the gut in inflammatory bowel disease and is overrepresented in a subset of patients that suffer from Crohn's disease that makes up about 25-50% of patients with Crohn's disease. In healthy individuals, Malassezia is only sporadically found in the gut or fecal samples. Hence, it appears that the presence of Malassezia species can be strongly correlated with inflammation and deregulation of the immune system and solutions to reduce the Malassezia burden might be desired.

Malassezia spp. and more specifically Malassezia restricta and Malassezia globosa are well documented to occur in conjunction with skin diseases such as atopic dermatitis and dandruff. Substantial evidence accumulated so far suggest that microbial communities on skin certainly have an influence on dandruff formation besides other physiological factors. Among all microorganisms, the strongest link between dandruff formation and microorganisms has been observed with Malassezia. Many comparative studies clarified that Malassezia numbers and the severity of dandruff correlate. In addition, bacteria can participate in increased severity of dandruff in addition to host-related physiological factors and Malassezia.

Another recent disclosure established the involvement of the common skin resident fungus Malassezia restricta in patients suffering from Crohn's disease that is also linked to the presence of an IBD-associated polymorphism in the gene for CARD9, a signaling adaptor important for anti-fungal defense. M. restricta elicits innate inflammatory responses largely through CARD9 and is recognized by Crohn's disease patient anti-fungal antibodies. This yeast elicits strong inflammatory cytokine production from innate cells harboring the IBD-linked polymorphism in CARD9 and exacerbates colitis via CARD9 in mouse models of disease. Collectively, these results suggest that targeting specific commensal fungi may be a therapeutic strategy for IBD and more specifically in the respective subpopulation of about 25-50% of Crohn's patients that are a carrier of this polymorphism.

Another recent disclosure established the involvement of the common skin resident fungus Malassezia spp and its dysbiosis appears to be involved in accelerated pancreatic oncogenesis. The fungus is strongly overrepresented in tumor samples of affected patients and its ablation was protective against progression of pancreatic cancer while repopulation with Malassezia accelerated oncogenesis that involves a deregulation of an immune defense pathway.

A therapeutic product and application will be desirable that can lead to the reduction of the abundance of Malassezia spec and hence in turn will contribute to reduction of the Malassezia spp associated phenotype/disease.

As with many antimicrobials or fungicides, many substances elicit only broader spectrum activities against many bacteria or fungi that may also contribute to the healthy phenotype and it is not desirable to also reduce these commensal or beneficial microorganisms. Therefore, there is a need for more precise antimicrobials or fungicides that target as narrow as possible only the specific microorganisms that is associated with the disease phenotype.

### SUMMARY OF THE INVENTION

In the present invention strains of bacteria and yeast were screened that elicit a specific growth inhibiting or killing function against Malassezia. Yeast strains in particular Kazachstania unispora was identified whose culture supernatant elicits a specific inhibition to Malassezia restricta and Malassezia globosa but with no appreciable activity against Malassezia sympodialis or Malassezia furfur, demonstrating a strain specific inhibiting activity for medical and cosmetic applications. The invention may be applied to the genuses outlined below, i.e bacterial, fungal, viral strains.

The invention relates in particular to a pharmaceutical composition comprising:
(i) a live strain from the genus of Kazachstania spp, Acetobacter spp. and/or Stenotrophomonas spp,
(ii) an attenuated strain from the genus of Kazachstania spp, Acetobacter spp. and/or Stenotrophomonas spp or,
(iii) a medically active substance isolated from a strain from the genus of Kazachstania spp, Acetobacter spp. and/or Stenotrophomonas spp, (i) to (iii) all for use in treating cancer associated with an infection or dysbiosis with Malassezia spp or pancreatic cancer.

The invention relates to a pharmaceutical composition comprising:
(i) a live strain from the genus of Kazachstania spp, Acetobacter spp. and/or Stenotrophomonas spp for use in treating pancreatic cancer associated with an infection or dysbiosis with Malassezia spp,
(ii) an attenuated strain from the genus of Kazachstania spp, Acetobacter spp. and/or Stenotrophomonas spp for use in treating pancreatic cancer associated with an infection or dysbiosis with Malassezia spp or,
(iii) a medically active substance isolated from a strain from the genus of Kazachstania spp, Acetobacter spp. and/or Stenotrophomonas spp. all of the above (i) to (iii) for use in treating pancreatic cancer associated with an infection or dysbiosis with Malassezia spp.

It is preferred if the cancer to be treated is associated with an infection or dysbiosis with Malassezia spp but also if the pancreatic cancer is associated with an infection or dysbiosis with Malassezia spp. In some instances, it may longer be possible to detect the Malassezia spp infection or dysbiosis and the treatment according to the invention is done preventively.

Medically active herein means, it displays the inhibitory activity of the strain itself or a higher inhibitory effect. Inhibitory means it inhibits growth, cell division, cell cycle reactions and/or viral, eukaryotic, yeast, fungal or bacterial physiological processes.

The medicament herein is used to treat a subject.

The term "Malassezia spp. associated disease" or "Malassezia spp. associated phenotype" as used herein may refer to a disease or a phenotype caused dybiosis of Malassezia spp or contributed by the presence of Malassezia spp. Further, it may be, for example, a chronic inflammatory disease such as spondyloarthritis or multiple sclerosis, a skin disease such as seborrheic dermatitis, or a bowel disease such as Crohn's disease, an oncogenic disease such as pancreatic cancer or even a combination of one or more of the above mentioned diseases.

Dysbiosis in this context means the under- or overrepresentation of a particular microorganism or several microorganisms at a specific microbial niche such as the skin, scalp, oral or vaginal epithelium or the gut and its subsections, or its presence in otherwise sterile body sites, such as the CNS, visceral organs, or the blood vessels. The meaning of microorganism includes bacteria, fungi, yeasts, archaea and viruses of these organisms but is not limited to this description as other terms such as mycobiota or fungome (the sum of all fungi at a body niche or system) might be overlapping with this definition and should not been seen as a limitation to the definition of microbiome or microbiota.

In the present invention, the term "prognosis" denotes a prediction of how a subject's (e.g. a patient's) medical condition will progress. This may include an estimation of the chance of recovery or the chance of an adverse outcome for said subject.

The term "subject" as used herein refers to a living human or non-human organism. Preferably herein the subject is a human subject. A subject treated by a method described herein, or by contact with or administration of a composition described herein can be a mammalian subject who can be a human subject, a non-human primate, a canine mammal, a felid mammal or any other mammal. A subject maybe a patient who is a mammalian patient for instance, a human patient, a non-human primate, a canine mammal, a felid mammal or any other mammalian patient.

In the present invention, the term therapy control denotes the attribution of a certain form of treatment, such as the administration of a medicament, to the state or progression of a subject's medical condition by measuring the level of a certain diagnostic marker or markers for said medical condition at various points of time, preferably before and after the treatment. In this way, it may be determined whether said treatment is adequate to treat said medical condition, or whether the therapy will have to be adjusted, e.g. by altering the dosage of the medicament, or will have to be replaced by another form of treatment, e.g. another medicament.

As used herein, the term "diagnosis" refers to the determination as to whether a subject is likely to present or develop a disease. The term "diagnosis" as used herein refers to methods by which the person skilled in the art can estimate and/or determine the probability ("a likelihood") of whether or not a subject is suffering from and/or further developing a disease, such as a skin disease. In the case of the present invention, "diagnosis" includes using the results of an assay.

As used herein, the term "prognosis" refers to the prediction of the likelihood of the disease attributable death or progression of a disease such as a skin disease, including recurrence, inflammation, infectious disease, auto immune disease, metabolic disease, oncogenic disease, genetic and non-genetic disease.

An infection in the sense of the present invention is also a dysbiosis. Dysbiosis (also called dysbacteriosis) is a term for a microbial imbalance or maladaptation on or inside the body, such as an impaired microbiota. For example, a part of the human microbiota, such as the skin flora, gut flora, or vaginal flora, can become deranged, with normally dominating species underrepresented and normally outcompeted or contained species increasing to fill the void. Dysbiosis is most commonly reported as a condition in the gastrointestinal tract, particularly during small intestinal bacterial overgrowth (SIBO) or small intestinal fungal overgrowth.

### DETAILED DESCRIPTION OF THE INVENTION

As outlined in the summary above the invention relates to a pharmaceutical composition comprising:
(i) a live strain from the genus of Kazachstania spp, Acetobacter spp. and/or Stenotrophomonas spp for use in treating pancreatic cancer associated with an infection or dysbiosis with Malassezia spp,
(ii) an attenuated strain from the genus of Kazachstania spp, Acetobacter spp. and/or Stenotrophomonas spp for use in treating pancreatic cancer associated with an infection or dysbiosis with Malassezia spp or,
(iii) a medically active substance isolated from a strain from the genus of Kazachstania spp, Acetobacter spp. and/or Stenotrophomonas spp. for use in treating pancreatic cancer associated with an infection or dysbiosis with Malassezia spp.

The invention relates in general to cancers that have been shown to be associated with an infection or dysbiosis with Malassezia spp. Hence it relates to treating pancreatic cancer associated with an infection or dysbiosis with Malassezia spp but also other cancers that are associated with an infection or dysbiosis with Malassezia spp.

The human digestive system consists of the gastrointestinal tract plus the accessory organs of digestion (the tongue, salivary glands, pancreas, liver, and gallbladder). Another recent disclosure established the involvement of the common skin resident fungus Malassezia spp and its dysbiosis appears to be involved in accelerated pancreatic oncogenesis.

Pancreatic ductal adenocarcinoma (PAD) is responsible for about 95% of all pancreas cancers. It is a devastating disease as being the fourth most leading cause for cancer-related death worldwide with a 5-year overall survival of less than 8%. The incidence of PAD is expected to rise 2fold over the next 10 years. Surgical resection followed by adjuvant chemotherapy is the only possibly curative therapy available, yet only 10-20% of PAD patients present with resectable PDAC stages, while the residual 80-90% show locally advanced, non-resectable stages. Systemic chemotherapy is commonly employed as first-line treatment in patients with non-resectable or borderline-resectable tumors. A recent 2019 publication clearly indicates that the skin commensal Malassezia spp promotes pancreatic oncogenesis (Ayuk at al,. 2019). Dysbiosis of the mycobiome shows a 3000fold overrepresentation and infiltration of Malassezia spp in the pancreatic tumor in patients and a PAD mouse model. It is demonstrated that following antifungal treatment, repopulation of the tumor with Malassezia but not other fungi accelerates oncogenesis. The supposed mechanism for accelerated oncogenesis appears to rely on ligation of mannose-binding lectin (MBL) that binds to glycans of the fungal wall and activates the complement cascade - an important pathway of the immune response, whereas deletion of MBL or C3 in the extra-tumoral compartment was protective against tumor growth. In conclusion, a Malassezia-specific antifungal treatment is supposed to support or increase efficacy of chemotherapy and other treatment approaches. Malassezia spp apparently infiltrates the pancreatic tissue through connections within the digestive system. Hence, it can be speculated that Malassezia spp can also play an important role in oncogenesis of other tissues of the digestive system such as the small and the large intestine, the gallbladder, and the liver.

Ideally, the claimed composition inhibits growth and/or cell division of Malassezia spp.

The invention also relates to a microbial strain selected from the genus of Kazachstania spp, Acetobacter spp. and/or Stenotrophomonas spp for use as a medicament,
(i) a cellular extract from a strain selected from the genus of Kazachstania spp, Acetobacter spp. and/or Stenotrophomonas spp for use as a medicament in treating pancreatic cancer associated with an infection or dysbiosis with Malassezia spp,
(ii) the supernatant from the cell culture from a strain selected from the genus of Kazachstania spp, Acetobacter spp. and/or Stenotrophomonas spp for use as a medicament in treating pancreatic cancer associated with an infection or dysbiosis with Malassezia spp or,
(iii) a medically active molecule or a medically active substance obtained from a strain selected from the genus of Kazachstania spp, Acetobacter spp. and/or Stenotrophomonas spp for use as a medicament in treating pancreatic cancer associated with an infection or dysbiosis with Malassezia spp.

Preferably, the strain is selected from the group of Kazachstania unispora, Acetobacter fabarum and Stenotrophomonas maltophila.

Preferably, the strain, extract, supernatant, molecule or substance inhibits growth and/or cell division of Malassezia spp.

A pharmaceutical dosage form of a tablet or capsule or parenteral which comprises a pharmaceutically effective amount of active ingredient is herein also a composition as claimed.

Preferably, the compositions of the invention are to be administered to the gastrointestinal tract in order to enable delivery to and/or partial or total colonization of the intestine with the bacterial or microbial strain of the invention. Generally, the compositions of the invention are administered orally, but they may be administered rectally, intranasally, or via buccal or sublingual routes. In some cases, the compositions are used to create a cream which may be administered to directly to the skin.

In certain embodiments, the compositions of the invention may be administered as a fluid, as a foam, as a spray or a gel.

In certain embodiments, the compositions of the invention may be administered as a suppository, such as a rectal suppository, for example in the form of a theobroma oil (cocoa butter), synthetic hard fat (e.g. suppocire, witepsol), glycero-gelatin, polyethylene glycol, or soap glycerin composition.

In certain embodiments, the composition of the invention is administered to the gastrointestinal tract via a tube, such as a nasogastric tube, orogastric tube, gastric tube, jejunostomy tube (J tube), percutaneous endoscopic gastrostomy (PEG), or a port, such as a chest wall port that provides access to the stomach, jejunum and other suitable access ports.

The compositions of the invention may be administered once, or they may be administered sequentially as part of a treatment regimen. In certain embodiments, the compositions of the invention are to be administered daily.

In certain embodiments of the invention, treatment according to the invention is accompanied by assessment of the subject's gut microbiota. Treatment may be repeated if delivery of and/or partial or total colonization with the strain of the invention is not achieved such that efficacy is not observed, or treatment may be ceased if delivery and/or partial or total colonization is successful and efficacy is observed.

In certain embodiments, the composition of the invention may be administered to a pregnant animal, for example a mammal such as a human in order to prevent an inflammatory or autoimmune disease developing in her child in utero and/or after it is born.

Diluents may be selected from the group comprising of mannitol, microcrystalline Cellulose, lactose, starch, dibasic calcium phosphate anhydrous, tribasic calcium phosphate, kaolin, sucrose, precipitated calcium carbonate, sorbitol, maltodextrin, powdered cellulose, micro crystalline cellulose and other materials known for such property. Diluents in the dosage form ranges from 0% to 78.0% by weight.

Lubricants may be selected from the group comprising of stearic acid, sodium stearyl fumarate, polyethylene glycol, magnesium stearate, calcium stearate, talc, zinc stearate, hydrogenated castor oil, silica, colloidal silica, cornstarch, calcium silicate, magnesium silicate, silicon hydrogel and other materials known for such property. Lubricants in the dosage form ranges from 0% to 3.0% by weight.

Binders may be selected from the group comprising of polyvinylpyrrolidone, hydroxypropyl methylcellulose, acacia, alginic acid, hydroxy propyl cellulose, carboxymethylcellulose sodium, compressible sugar, ethylcellulose, gelatin, liquid glucose, methylcellulose, pregelatinized starch and other materials known to one of ordinary skill in the art. Binders in the dosage form ranges from 0% to 5.0% by weight.

Glidants may be selected from the group comprising of colloidal silicon dioxide, colloidal silica, cornstarch, talc, calcium silicate, magnesium silicate, colloidal silicon, silicon hydrogel and other materials known for such property. Glidants in the dosage form ranges from 0% to 2.0% by weight.

The cosmetic composition according to the invention may comprise from 0.0001% to 1%, preferably from 0.001% to 0.4% and more preferably from 0.002% to 0.15% of the additional strain or substance by weight of the composition.

The pharmaceutical composition may optionally be coated with functional and/or non-functional layers comprising film-forming polymers, if desired. Examples of film-forming polymers include ethylcellulose, hydroxypropyl methylcellulose, hydroxypropylcellulose, polyvinyl alcohol, polyvinyl acetate methylcellulose, carboxymethyl cellulose, hydroxymethylcellulose, hydroxyethylcellulose, cellulose acetate, hydroxypropyl methylcellulose phthalate, cellulose acetate phthalate, cellulose acetate trimellitate; waxes; methacrylic acid polymers and the like. Alternatively, commercially available coating compositions comprising film-forming polymers marketed under various trade names, such as Opadry may also be used for coating. Film-forming polymer in the dosage form ranges from 1.5% to 4.0% by weight.

Cosmetic compositions according to the present invention may further comprise a hydrophobic vitamin component. The hydrophobic vitamin component may comprise any vitamin that fulfils the definition of "hydrophobic" provided herein. The hydrophobic vitamin component may advantageously comprise vitamin E or hydrophobic derivatives thereof, preferably tocopheryl nicotamide, vitamin D or derivatives thereof, or mixtures thereof. The cosmetic composition according to the invention may comprise from 0.01% to 10%, preferably from 0.1% to 9%, more preferably from 0.5% to 5% of at least one hydrophobic vitamin component by weight of the composition.

A variety of additional optional ingredients may be incorporated into the compositions of the present invention. Non-limiting examples of these additional ingredients include additional skin care actives such as bisabolol, dialkanoyl hydroxyproline compounds, farnesol, flavonoids, guanidine (e.g., amino guanidine), N-acyl amino acid compounds, peptides, phytantriol, phytosterols, salicylic compounds, urea as well as compounds such as anti-acne compounds (e.g. resorcinol, erythromycin); antioxidants compounds (e.g., phytosterols, lipoic acid); skin soothing and healing agents; anti-wrinkle/anti-atrophy actives; conditioning agents; anti-inflammatory agents; skin lightening agents; antimicrobial/antibacterial/antifungal actives; chelators and sequestrants; and agents suitable for aesthetic purposes such as essential oils, fragrances, skin sensates, opacifiers, aromatic compounds (e.g., clove oil, menthol, camphor, eucalyptus oil, and eugenol), preservatives, or mixtures thereof.

Preferably it relates to a pharmaceutical composition comprising:
(iv) a live strain from the genus of Kazachstania spp, Acetobacter spp. and/or Stenotrophomonas spp,
(v) an attenuated strain from the genus of Kazachstania spp, Acetobacter spp. and/or Stenotrophomonas spp or,
(vi) a medically active substance isolated from a strain from the genus of Kazachstania spp, Acetobacter spp. and/or Stenotrophomonas spp.
for use as a medicament.

Generally, the composition of the invention comprises microorganisms. In preferred embodiments of the invention, the composition is formulated in freeze-dried form. For example, the composition of the invention may comprise granules or gelatin capsules, for example hard gelatin capsules, comprising a microbial or a bacterial strain of the invention.

Preferably, the composition of the invention comprises lyophilized microorganisms. Lyophilization of bacteria and/or yeast is a well-established procedure and relevant guidance is available.

Alternatively, the composition of the invention may comprise a live, active microbial culture.

In some embodiments, the microorganism strain in the composition of the invention has not been inactivated, for example, has not been heat-inactivated. In some embodiments, the microbial strain in the composition of the invention has not been killed, for example, has not been heat-killed. In some embodiments, the microbial strain in the composition of the invention has not been attenuated, for example, has not been heat-attenuated. For example, in some embodiments, the microbial strain in the composition of the invention has not been killed, inactivated and/or attenuated. For example, in some embodiments, the microbial strain in the composition of the invention is live. For example, in some embodiments, the microbial strain in the composition of the invention is viable. For example, in some embodiments, the microbial strain in the composition of the invention is capable of at least partially or totally colonizing the intestine and/or the skin. For example, in some embodiments, the microbial strain in the composition of the invention is viable and capable of at least partially or totally colonizing the intestine and/or the skin.

In some embodiments, the composition comprises a mixture of live microbial strains and microbial strains that have been killed.

In preferred embodiments, the composition of the invention is encapsulated to enable delivery of the microbial strain to the intestine. Encapsulation protects the composition from degradation until delivery at the target location through, for example, rupturing with chemical or physical stimuli such as pressure, enzymatic activity, or physical disintegration, which may be triggered by changes in pH. Any appropriate encapsulation method may be used. Exemplary encapsulation techniques include entrapment within a porous matrix, attachment or adsorption on solid carrier surfaces, self-aggregation by flocculation or with cross-linking agents, and mechanical containment behind a microporous membrane or a microcapsule. Guidance on encapsulation that may be useful for preparing compositions of the invention is available in.

The composition may be administered orally and may be in the form of a tablet, capsule or powder.

The composition may be formulated as a probiotic.

A composition of the invention includes a therapeutically effective amount of a microbial strain of the invention. A therapeutically effective amount of a microbial strain is sufficient to exert a beneficial effect upon a subject. A therapeutically effective amount of a microbial strain may be sufficient to result in delivery to and/or partial or total colonization of the subject's intestine and/or skin.

A suitable daily dose of the microorganism, for example for an adult human, may be from about 1×10³ to about 1×10¹¹ colony forming units (CFU); for example, from about 1×10⁷ to about 1×10¹⁰ CFU; in another example from about 1×10⁶ to about 1×10¹⁰ CFU. In certain embodiments, the composition contains the bacterial strain in an amount of from about 1×10⁶ to about 1×10¹¹ CFU/g, respect to the weight of the composition; for example, from about 1×10⁸ to about 1×10¹⁰ CFU/g. The dose may be, for example, 1 g, 3 g, 5 g, and 10 g of the composition.

Typically, a probiotic, such as the composition of the invention, is optionally combined with at least one suitable prebiotic compound. A prebiotic compound is usually a non-digestible carbohydrate such as an oligo- or polysaccharide, or a sugar alcohol, which is not degraded or absorbed in the upper digestive tract. Known prebiotics include commercial products such as inulin and transgalacto-oligosaccharides.

In certain embodiments, the probiotic composition of the present invention includes a prebiotic compound in an amount of from about 1 to about 30% by weight, respect to the total weight composition, (e.g. from 5 to 20% by weight). Carbohydrates may be selected from the group consisting of: fructo-oligosaccharides (or FOS), short-chain fructo-oligosaccharides, inulin, isomalt-oligosaccharides, pectins, xylo-oligosaccharides (or XOS), chitosan-oligosaccharides (or COS), beta-glucans, arable gum modified and resistant starches, polydextrose, D-tagatose, acacia fibers, carob, oats, and citrus fibers. In one aspect, the prebiotics are the short-chain fructo-oligosaccharides (for simplicity shown herein below as FOSs-c.c); said FOSs-c.c. are not digestible carbohydrates, generally obtained by the conversion of the beet sugar and including a saccharose molecule to which three glucose molecules are bonded.

The compositions of the invention may comprise pharmaceutically acceptable excipients or carriers. Acceptable carriers or diluents for therapeutic use are well known. Examples of suitable carriers include lactose, starch, glucose, methyl cellulose, magnesium stearate, mannitol, sorbitol and the like. Examples of suitable diluents include ethanol, glycerol and water. The choice of pharmaceutical carrier, excipient or diluent can be selected with regard to the intended route of administration and standard pharmaceutical practice. The pharmaceutical compositions may comprise as, or in addition to, the carrier, excipient or diluent any suitable binder(s), lubricant(s), suspending agent(s), coating agent(s), solubilizing agent(s). Examples of suitable binders include starch, gelatin, natural sugars such as glucose, anhydrous lactose, free-flow lactose, beta-lactose, corn sweeteners, natural and synthetic gums, such as acacia, tragacanth or sodium alginate, carboxymethyl cellulose and polyethylene glycol. Examples of suitable lubricants include sodium oleate, sodium stearate, magnesium stearate, sodium benzoate, sodium acetate, sodium chloride and the like. Preservatives, stabilizers, dyes and even flavoring agents may be provided in the pharmaceutical composition. Examples of preservatives include sodium benzoate, sorbic acid and esters of p-hydroxybenzoic acid. Antioxidants and suspending agents may be also used.

The compositions of the invention may be formulated as a food product. For example, a food product may provide nutritional benefit in addition to the therapeutic effect of the invention, such as in a nutritional supplement. Similarly, a food product may be formulated to enhance the taste of the composition of the invention or to make the composition more attractive to consume by being more similar to a common food item, rather than to a pharmaceutical composition. In certain embodiments, the composition of the invention is formulated with a nutritious product such as a milk-based product. The term "milk-based product" means any liquid or semi-solid milk- or whey-based product having a varying fat content. The milk-based product can be, e.g., cow's milk, goat's milk, sheep's milk, skimmed milk, whole milk, milk recombined from powdered milk and whey without any processing, or a processed product, such as yoghurt, curdled milk, curd, sour milk, sour whole milk, butter milk and other sour milk products. Another important group includes milk beverages, such as whey beverages, fermented milks, condensed milks, infant or baby milks; flavored milks, ice cream; milk-containing food such as sweets.

The invention relates also to a medicament and as such to a pharmaceutical composition comprising:
(i) a live strain from the genus of Kazachstania spp, Acetobacter spp. and/or Stenotrophomonas spp,
(ii) an attenuated strain from the genus of Kazachstania spp, Acetobacter spp. and/or Stenotrophomonas spp or,
(iii) a medically active substance isolated from a strain from the genus of Kazachstania spp, Acetobacter spp. and/or Stenotrophomonas spp.
for use as a medicament.

Preferably, this is pancreatic cancer, as outlined above.

Preferably, the oncogenesis of the digestive system is associated with an infection with Malassezia spp. More preferably pancreatic ductal adenocarcinoma is associated with an infection with Malassezia spp.cPreferably, the strain, extract, supernatant, molecule or substance is for use in treating an oncogenesis of the digestive system which is selected from the group of pancreatic ductal adenocarcinoma, small and large intestine, gallbladder and liver and more preferably for use in treating an oncogenesis of the pancreatic ductal andenocarcinoma.

Preferably the invention relates to a a pharmaceutical composition comprising:
(i) a live strain from the genus of Kazachstania spp, Acetobacter spp. and/or Stenotrophomonas spp,
(ii) an attenuated strain from the genus of Kazachstania spp, Acetobacter spp. and/or Stenotrophomonas spp or,
(iii) a medically active substance isolated from a strain from the genus of Kazachstania spp, Acetobacter spp. and/or Stenotrophomonas spp. for use in treating a skin disease and/or a disease associated with an infection with Malassezia spp.

The skin disease is preferably selected from the group of seborrheic dermatitis, psoriasis, acne and dandruff.

The compositions for use in accordance with the invention may or may not require marketing approval.

In certain embodiments, the invention provides the above pharmaceutical composition, wherein said microbial strain is lyophilized. In certain embodiments, the invention provides the above pharmaceutical composition, wherein said microbial strain is spray dried. In certain embodiments, the invention provides the above pharmaceutical composition, wherein the microbial strain is lyophilized or spray dried and wherein it is live. In certain embodiments, the invention provides the above pharmaceutical composition, wherein the microbial strain is lyophilized or spray dried and wherein it is viable. In certain embodiments, the invention provides the above pharmaceutical composition, wherein the microbial strain is lyophilized or spray dried and wherein it is capable of at least partially or totally colonizing the intestine and/or the skin. In certain embodiments, the invention provides the above pharmaceutical composition, wherein the microbial strain is lyophilized or spray dried and wherein it is viable and capable of at least partially or totally colonizing the intestine.

The strain is preferably selected from the group of Kazachstania unispora, Acetobacter fabarum and Stenotrophomonas maltophila.

Ideally, the composition inhibits growth and/or cell division of Malassezia spp.

The invention also relates to
(i) a microbial strain selected from the genus of Kazachstania spp, Acetobacter spp. and/or Stenotrophomonas spp for use as a medicament,
(ii) a cellular extract from a microbial strain selected from the genus of Kazachstania spp, Acetobacter spp. and/or Stenotrophomonas spp for use as a medicament,
   the supernatant from the cell culture from a strain selected from the genus of Kazachstania spp, Acetobacter spp. and/or Stenotrophomonas spp for use as a medicament or,
(iii) a medically active molecule or a medically active substance obtained from a strain selected from the genus of Kazachstania spp, Acetobacter spp. and/or Stenotrophomonas spp for use as a medicament, all of the above for treating pancreatic cancer.

Ideally, the strain is selected from the group of Kazachstania unispora, Acetobacter fabarum and Stenotrophomonas maltophila.

Preferably, the strain, extract, supernatant, molecule or substance inhibits growth and/or cell division of Malassezia spp.

### FIGURE CAPTIONS

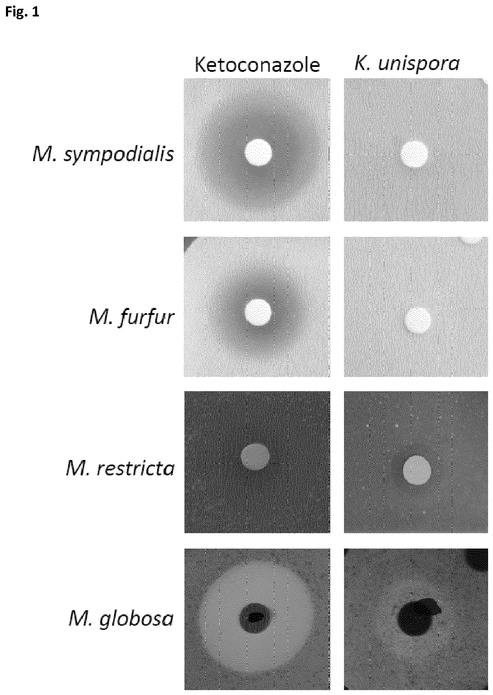

Fig. 1 | Inhibition assays of the fungicide ketoconazole as positive control for inhibition and *Kazachstania unispora* supernatant on *M. sympodialis, M. furfur, M. restricta* and *M*. *globosa.* Inhibition assays were performed using the disc diffusion method. Discs were soaked with ketoconazole (1mg/ml) or *K. unispora* supernatant and placed on agar plates containing a soft layer inoculated with a single culture of the different *Malassezia* species to be tested. On the left column of pictures, halo with no growth indicates that ketoconazole inhibits growth of all 4 *Malassezia* species tested. On the right column, an inhibition halo is only seen on *M*. *restricta* and *M*. *globosa* agar plates, indicating that *K. unispora* inhibits growth of those two species but that it does not inhibit growth of *M. sympodialis* and *M. furfur.*

Fig. 1 | Inhibition assays of *M*. *restricta* and *M. globose* by growth of Acetobacter fabarum. Discs were soaked with ketoconazole (1mg/ml in DMSO) or exponentially growing Acetobacter fabarum culture and placed on agar plates containing a soft layer inoculated with a single culture of the different *Malassezia* species to be tested. In the upper panel, inhibition of Malassezia globosa is shown; in the bottom panel, Malassezia restricta is shown. The halo with no growth indicates that ketoconazole inhibits growth of both *Malassezia* species tested. An inhibition halo is also seen around the disk imbued with growing culture of Acetobacter fabarum for both species of Malasseiza tested.

### EXAMPLES

### Example 1

In order to identify strains inhibiting Malassezia growth, inhibition assays were performed using the disc diffusion method. This method consists in disposing discs imbibed with potential inhibitory substances on Petri plates containing a strain of interest. After optimal growth of the strain of interest, growth inhibition can be observed around discs soaked with substances having an inhibitory effect. Potential inhibitory substances tested in this experiment are supernatants obtained after individually culturing a collection of fungal and bacterial strains. After an incubation of 10 days in their optimal broth, each culture was centrifuged and the supernatant was filtered and further stored at -20°C. Ketoconazole, a fungicide, was used as a positive control for Malassezia growth inhibition. It was diluted in dimethyl sulfoxide at 1mg/ml. The collection of supernantants was tested on the following species from the genus Malassezia: Malassezia restricta, Malassezia globosa, Malassezia furfur and Malassezia sympodialis. All strains were streaked from -80°C stock on Modified Leeming and Notman (MLN) agar plates and incubated 10 days at 30°C. A 5ml liquid culture of each species was made in MLN broth and incubated at 30°C for 2 days for M. furfur and M. sympodialis and 5 days for M. restricta and M. globosa. To allow a lawn growth of the different Malassezia strains tested, Petri plates were prepared as follows: a bottom layer of 50ml Modified Sabouraud Dextrose (MSD) agar was poured in Petri plates. The soft agar, constituting the top layer, was a mix of 12,5ml MSD agar, 10,5ml MLN broth and 2ml of liquid culture. Plates were allowed to dry for one hour, then, 6mm-diameter WhatmanTM discs were soaked with 15ul of each thawed supernatants and ketoconazole and placed on the soft agar plate containing the solidified Malassezia monoculture layer. After an incubation of 5 days, inhibition area could be easily observed around discs. As expected, ketoconazole inhibited all 4 Malassezia species tested in this experiment. In addition, one supernantant was able to inhibit the growth of Malassezia globosa and Malassezia restricta while having no effect on Malassezia furfur and Malassezia sympodialis (see figure 1). The strain from which the inhibitory supernatant has been produced was further sequenced using the Sanger technology. The internal transcribed spacer (ITS) of this strain was taxonomically assigned to the yeast Kazachstania unispora (BLASTn, 99% identity, 99% query cover, e-value 0.00).

### Example 2

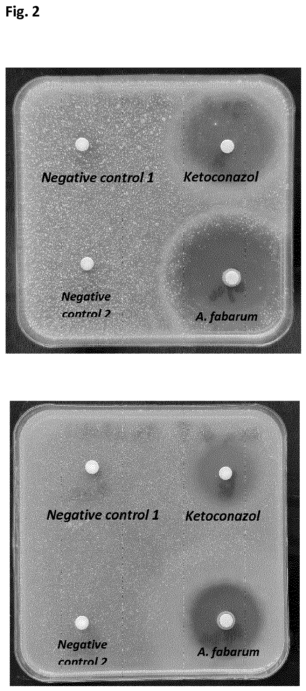

In order to identify strains whose growth inhibits Malassezia, inhibition assays were performed using a modified disc diffusion method. This method is similar to the classical disk diffusion method that consists in depositing discs imbibed with potential inhibitory substances on Petri plates containing a strain of interest, except that the disks were imbibed with a living culture of an Acetobacter strain. After optimal growth of the strain of interest, growth inhibition can be observed around discs on which the growth of inhibitory bacteria has occurred. Ketoconazole (at 1 mg/mL in DMSO) was used as a positive control for Malassezia growth inhibition. The effect of growth of Acetobacter was tested on Malassezia restricta and Malassezia globosa. All Malassezia strains were streaked from -80°C stock on Modified Leeming and Notman (MLN) agar plates and incubated 10 days at 30°C. A 5ml liquid culture of each species was made in MLN broth and incubated at 30°C for 5. To allow a lawn growth of the different Malassezia strains tested, Petri plates were prepared as follows: a bottom layer of 50ml Modified Sabouraud Dextrose (MSD) agar was poured in Petri plates. The soft agar, constituting the top layer, was a mix of 12,5ml MSD agar, 10,5ml MLN broth and 2ml of liquid culture. Plates were allowed to dry for one hour, then, 6mm-diameter WhatmanTM discs were soaked with 15ul of exponentially growing cultures of Acetobacter and placed on the soft agar plate containing the solidified Malassezia monoculture layer. After an incubation of 5 days, inhibition area could be easily observed around discs (see below). As expected, ketoconazole inhibited both Malassezia restricta and Malassezia globose. In addition, one supernantant was able to inhibit the growth of Malassezia globosa and Malassezia restricta (see figure 2).

## Claims

1. A pharmaceutical composition comprising:
(iv) a live strain from the genus of Kazachstania spp, Acetobacter spp. and/or Stenotrophomonas spp for use in treating a pancreatic cancer, or a cancer associated with an infection or dysbiosis with Malassezia spp,
(v) an attenuated strain from the genus of Kazachstania spp, Acetobacter spp. and/or Stenotrophomonas spp for use in treating pancreatic cancer or a cancer associated with an infection or dysbiosis with Malassezia spp or,
(vi) a medically active substance isolated from a strain from the genus of Kazachstania spp, Acetobacter spp. and/or Stenotrophomonas spp for use in treating pancreatic cancer or a cancer associated with an infection or dysbiosis with Malassezia spp.

2. A pharmaceutical composition according to claim 1, wherein the strain is selected from the group of Kazachstania unispora, Acetobacter fabarum and Stenotrophomonas maltophila.

3. A pharmaceutical composition according to claims 1 or 2, wherein the composition inhibits growth and/or cell division of Malassezia spp.

4. A pharmaceutical composition according to claims 1 to 3, wherein the pancreatic cancer is a pancreatic ductal andenocarcinoma and/or wherein it is associated with an infection or dysbiosis with Malassezia spp.

5. A pharmaceutical composition according to claims 1 to 4, wherein the cancer is selected from the group of pancreatic ductal adenocarcinoma, small intestine cancer, large intestine cancer, gallbladder cancer, and liver cancer.

6. A microbial strain selected from the genus of Kazachstania spp, Acetobacter spp. and/or Stenotrophomonas spp for use as a medicament,
(i) a cellular extract from a strain selected from the genus of Kazachstania spp, Acetobacter spp. and/or Stenotrophomonas spp for use as a medicament in treating pancreatic cancer associated with an infection or dysbiosis with Malassezia spp,
(ii) the supernatant from the cell culture from a strain selected from the genus of Kazachstania spp, Acetobacter spp. and/or Stenotrophomonas spp for use as a medicament in treating pancreatic cancer associated with an infection or dysbiosis with Malassezia spp or,
(iii) a medically active molecule or a medically active substance obtained from a strain selected from the genus of Kazachstania spp, Acetobacter spp. and/or Stenotrophomonas spp for use as a medicament in treating pancreatic cancer associated with an infection or dysbiosis with Malassezia spp.

7. A strain according to claim 6, wherein the strain is selected from the group of Kazachstania unispora, Acetobacter fabarum and Stenotrophomonas maltophila.

8. A strain, extract, supernatant, molecule or substance according to claims 6 or 7, wherein the strain, extract, supernatant, molecule or substance inhibits growth and/or cell division of Malassezia spp.

9. A strain, extract, supernatant, molecule or substance according to claims 6 to 8, wherein the pancreatic cancer is a pancreatic ductal andenocarcinoma.

10. A strain, extract, supernatant, molecule or substance according to claims 6 to 8, wherein the cancer is selected from the group of pancreatic ductal adenocarcinoma, small intestine cancer, large intestine cancer, gallbladder cancer, and liver cancer.
